# EUROPEAN PATENT SPECIFICATION

(11) **EP 4 279 605 B1**
(45) Date of publication and mention of the grant of the patent: **21.10.2026**
(21) Application number: 21919566.6
(22) Date of filing: 22.11.2021
(51) Int. Cl.: C12Q 1/06, G01N 15/06, C12M 1/34, G01N 15/01, G01N 15/075, C12M 1/00, G01N 33/487

(54) **VIABLE CELL COUNT MEASUREMENT METHOD AND VIABLE CELL COUNT MEASUREMENT DEVICE**
MESSVERFAHREN UND MESSVORRICHTUNG FÜR ANZAHL LEBENSFÄHIGER ZELLEN
PROCÉDÉ DE MESURE DE NOMBRE DE CELLULES VIABLES ET DISPOSITIF DE MESURE DE NOMBRE DE CELLULES VIABLES

(30) Priority: 12.01.2021 JP 2021003012
(43) Date of publication of application: 22.11.2023
(60) Divisional application: 26202654.5 / 4 826 381
(73) Proprietor: Hitachi Plant Services Co., Ltd., Tokyo 110-0015 (JP)
(72) Inventor: SHIBUYA, Keisuke, Tokyo 100-8280 (JP); OKA, Kenichiro, Tokyo 100-8280 (JP)
(74) Representative: Mewburn Ellis LLP
(86) International application number: PCT/JP2021/042836
(87) International publication number: WO 2022/153675

(56) References cited:
- EP-A1- 3 581 646
- WO-A1-2019/142590
- US-A1- 2008 312 843
- ZHAO LIANG ET AL: "Advances in process monitoring tools for cell culture bioprocesses", ENGINEERING IN LIFE SCIENCES, vol. 15, no. 5, 24 April 2015 (2015-04-24), DE, pages 459 - 468, XP055903694, ISSN: 1618-0240, DOI: 10.1002/elsc.201500006

## Description

### Technical Field

The present invention relates to a living cell counting method and a living cell counting device that use a measurement result of capacitance, which varies depending on whether cells are living or dead, to calculate the number of living cells and the like.

### Background Art

In the related art, a method for producing a useful substance by culturing cells has been used in fields such as a brewing industry, a food industry, a chemical industry, and a pharmaceutical industry. For example, as biopharmaceutical products such as antibody drugs, many products containing substances produced by cells as main ingredients have been developed. Such biopharmaceutical products are produced by culturing cells in a culture solution and separating and purifying a target substance secreted in the culture solution.

In production of the useful substance using cells, culture control for efficiency of production and appropriate quality control related to products are required. During the culture, since it is desired to keep the number of cells large while avoiding an increase in impurities caused by cell death or the like, it is important to monitor the number of cells and whether cells are living or dead in real time.

In the related art, as a culture method suitable for production of the useful substance, a feeding culture method, a continuous culture method, and the like are known. In these culture methods, culture is performed while maintaining a concentration of nutrients and a culture environment. Since it is necessary to control an addition amount of glutamine or the like according to a specific growth rate of cells, monitoring the number of living cells is particularly important.

Generally, as a method for measuring the number of cells, there are a method in an off-line manner and a method in an in-line manner. The cell counting method in an off-line manner is for cells sampled from a culture system. On the other hand, the cell counting method in an in-line manner is suitable for real-time monitoring of cells being cultured in a culture system and reduction of contamination.

Examples of the cell counting method in an off-line manner include a method in which a culture solution intermittently sampled from a culture system is observed with a microscope, and cells visually observed in a microscope field of view are counted with a hemocytometer, and a method in which cells in a microscopic image are automatically counted by image processing. According to these methods, it is possible to determine whether cells are living or dead by staining the cells with trypan blue, and it is possible to individually count living cells and dead cells.

Examples of the cell counting method in an in-line manner include a method for estimating the number of cells based on an oxygen consumption amount in a culture solution and a method for estimating the number of cells based on capacitance, impedance, or dielectric constant of the culture solution. In the method based on the oxygen consumption amount, living cells can be quantified on the premise that a decrease in dissolved oxygen concentration is caused by oxygen consumption of the living cells. In the method based on the capacitance or the like, a response to an electric field is different between the living cells and the dead cells, and the living cells are quantified based on the premise that only the living cells generate polarization inside and outside the cells.

PTL 1 describes a cell inspection device that estimates the number of living cells with high accuracy based on impedance of a culture solution. The cell inspection device includes: an impedance sensor that measures the impedance of the culture solution; a storage unit in which a predetermined period in a culture period from the start of cell culture to death is classified into a plurality of periods, and a coefficient for estimating the number of living cells that live in the culture solution during the predetermined period is stored using the impedance for each of the plurality of classified periods; and a living cell estimation unit that acquires the impedance and estimates the number of living cells using at least one of the coefficients for each period stored in the storage unit for the impedance.

US2008/312843A1 discloses a process for on-line and in situ counting of cells in a biological culture medium.

EP3581646A1 discloses a method for producing and/or purifying measles virus particles from a sample.

ZHAO LIANG ET AL: "Advances in process monitoring tools for cell culture bioprocesses", ENGINEERING IN LIFE SCIENCES, vol. 15, no. 5, 24 April 2015, pages 459-468, XP055903694, ISSN: 1618-0240, discloses advances in process monitoring tools for cell culture bioprocesses.

### Citation List

### Patent Literature

PTL 1: JP2019-124594A

### Summary of Invention

### Technical Problem

There is a demand for a technique for quantifying the number of living cells contained in a cell suspension with high accuracy for a culture solution or the like for producing a useful substance. However, the related-art method in an off-line manner that requires sampling and the related-art method in an in-line manner based on the capacitance or the like have the following problems.

In the method in an off-line manner, it is possible to determine whether cells are living or dead by staining the cells with trypan blue, but it is necessary to sample the culture solution outside a culture tank. In such a method, there is a risk that the inside of the culture tank is contaminated with germs when the culture tank is temporarily opened. Since staining the cells with trypan blue is invasive to living cells, it is difficult to use the method in an in-line manner.

In the method for performing sampling, a sampling pipe need to be sterilized with high-temperature, high-pressure steam, or the like in order to prevent germs from entering the culture tank after sampling. At the time of steam sterilization, a time for heating the inside of the pipe, a sterilization time, and a time for cooling the inside of the pipe to room temperature are required. Since the sterilization time is usually 20 minutes or more, a sampling interval is about 1 hour or more, and real-time monitoring cannot be properly performed.

In the method in an in-line manner, since the culture solution is not sampled, problems such as long sterilization time do not occur, but there are several problems depending on a measurement method.

In the method based on the oxygen consumption amount, since it is necessary to stop the control of an oxygen amount at the time of measuring the dissolved oxygen concentration, there is a problem that the culture solution is temporarily in a state of a low oxygen concentration and production efficiency and quality of the useful substance deteriorate. In the method based on the capacitance or the like in the related art, since the living cells and the dead cells with cell membranes left cannot be distinguished from each other, and the dead cells with the cell membranes left may also be quantified, there is a problem that the number of living cells cannot be measured with high accuracy.

Therefore, an object of the invention is to provide a living cell counting method and a living cell counting device that are capable of measuring the number of living cells included in cells with high accuracy by non-invasive means with a low risk of contamination.

### Solution to Problem

In order to solve the above problems, a living cell counting method according to the invention includes: a step of measuring an amount of suspensoids contained in a cell suspension; a step of measuring capacitance of the cell suspension which varies depending on whether cells are living or dead; and a step of calculating one or more of the number of living cells, the number of dead cells, and a survival rate of cells that are contained in the cell suspension, based on capacitance per unit of living cells obtained in advance, capacitance per unit of dead cells obtained in advance, the amount of the suspensoids, and the capacitance of the cell suspension.

A living cell counting device according to the invention includes: a turbidity sensor configured to measure an amount of suspensoids contained in a cell suspension; a measuring instrument configured to measure capacitance of the cell suspension which varies depending on whether cells are living or dead; a storage unit configured to store data of the capacitance per unit of living cells and the capacitance per unit of dead cells; and a calculation unit configured to calculate one or more of the number of living cells, the number of dead cells, and a survival rate of cells that are contained in the cell suspension, based on the capacitance per unit of living cells, the capacitance per unit of dead cells, the amount of the suspensoids, and the capacitance of the cell suspension.

### Advantageous Effects of Invention

According to the invention, it is possible to provide a living cell counting method and a living cell counting device that are capable of measuring the number of living cells included in cells with high accuracy by non-invasive means with a low risk of contamination.

### Brief Description of Drawings

[FIG. 1] FIG. 1 is a diagram showing a configuration of a living cell counting device according to an embodiment of the invention.
[FIG. 2A] FIG. 2A is a diagram showing an example of a turbidity sensor according to a transmitted light measurement method.
[FIG. 2B] FIG. 2B is a partially enlarged view of FIG. 2A.
[FIG. 3] FIG. 3 is a diagram showing an example of a relation between turbidity of a cell suspension and the number of cells.
[FIG. 4] FIG. 4 is a diagram showing an example of a measuring instrument using a reflection transmission method.
[FIG. 5] FIG. 5 shows an example of a relation between capacitance of the cell suspension and the number of living cells.
[FIG. 6] FIG. 6 is a diagram showing a batch culture apparatus including the living cell counting device.
[FIG. 7] FIG. 7 is a diagram showing a fed-batch culture apparatus including the living cell counting device.
[FIG. 8] FIG. 8 is a diagram showing a chemostat culture apparatus including the living cell counting device.
[FIG. 9] FIG. 9 is a diagram showing a perfusion culture apparatus including the living cell counting device.
[FIG. 10] FIG. 10 is a diagram showing the living cell counting device used in Example 1 in which batch culture is performed.
[FIG. 11] FIG. 11 is a diagram showing measurement results of the number of living cells in Example 1 in which the batch culture is performed.
[FIG. 12] FIG. 12 is a diagram showing a deviation of the measurement results in Example 1 from that obtained by a method in the related art.
[FIG. 13] FIG. 13 is a diagram showing a living cell counting device used in Example 2 in which perfusion culture is performed.
[FIG. 14] FIG. 14 is a diagram showing measurement results of the number of living cells in Example 2 in which the perfusion culture is performed.

### Description of Embodiments

Hereinafter, a living cell counting method and a living cell counting device according to an embodiment of the invention will be described with reference to the drawings. In the following drawings, common components are denoted by the same reference numerals, and repetitive descriptions thereof are omitted.

A living cell counting method according to the present embodiment relates to a measuring method for quantifying the number of living cells contained in a cell suspension. In this living cell counting method, the cell suspension such as a culture solution is to be measured, and one or more of the number of living cells, the number of dead cells, and a survival rate of the cells contained in a cell suspension are obtained by measurement and calculation.

In the living cell counting method according to the present embodiment, an amount of suspensoids contained in the cell suspension is measured, and capacitance of the cell suspension caused by a polarization of a cell membrane is measured. When these measurements are combined, a measurement result of the capacitance can be converted into the amount of the suspensoids each correlating with the number of cells. Therefore, a measurement result of the number of cells or a cell concentration can be obtained based on the measurement result of the capacitance.

In the living cell counting method according to the present embodiment, contribution of each of the living cells and the dead cells to the capacitance of the cell suspension is considered. Specifically, capacitance per unit of living cells obtained in advance and capacitance per unit of dead cells obtained in advance are incorporated into simultaneous model equations of a total number of cells contained in the cell suspension and the capacitance of the cell suspension, and the number of living cells, the number of dead cells, and the survival rate of the cells that are contained in the cell suspension are calculated.

In general, when an electric field is applied to the cell suspension, a cell having a cell membrane acts as a dielectric, and thus polarization occurs between the inside and the outside of the cell with the cell membrane interposed therebetween. Since individual cells having a cell membrane generate capacitance due to the polarization, the number of cells having a cell membrane can be obtained from the measured capacitance of the cell suspension based on a correlation between the capacitance and the number of cells.

In a general cell counting method in the related art, the number of cells is estimated from capacitance, impedance, or dielectric constant. Such a general method in the related art enables quantification of the number of living cells, but is based on the premise that the polarization occurs only in the living cells. In the actual cell suspension, since cells are dead with cell membranes left or the like, dead cells in which polarization occurs may be included. In the general method in the related art, there is a problem that since such dead cells are also counted as living cells, the number of living cells cannot be measured with high accuracy.

On the other hand, when the capacitance per unit of living cells and the capacitance per unit of dead cells are obtained in advance, these data are incorporated into the simultaneous model equations of the total number of cells contained in the cell suspension and the capacitance of the cell suspension, and the calculation is performed, it is possible to obtain capacitance of total living cells or the number of living cells obtained by synthesizing all the living cells and capacitance of total dead cells or the number of dead cells obtained by synthesizing all the dead cells. Therefore, even if the survival rate of the cells is unknown, the number of living cells contained in the cell suspension can be obtained with high accuracy.

In the living cell counting method according to the present embodiment, types of cells to be measured for the number of living cells are not particularly limited as long as the total number of cells can be measured as the amount of the suspensoids. Cells to be measured may be animal cells, insect cells, plant cells, microalgae, cyanobacteria, bacteria, yeast, fungi, algae, or the like. The cells to be measured are preferably cells that produce various antibodies such as a human antibody, a humanized antibody, a chimeric antibody, and a mouse antibody, various physiologically active substances, and various useful substances useful as pharmaceutical raw materials, chemical raw materials, food raw materials, and the like.

Hereinafter, the living cell counting device according to an embodiment of the invention will be described together with details of the living cell counting method with reference to the drawings.

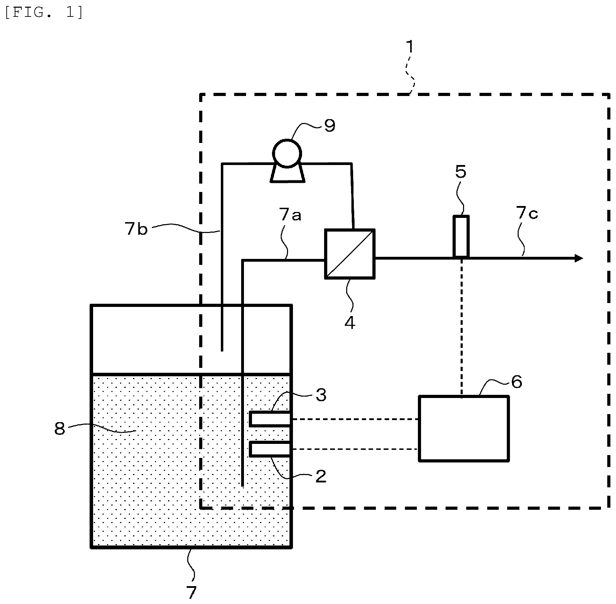

FIG. 1 is a diagram showing a configuration of the living cell counting device according to an embodiment of the invention.

As shown in FIG. 1, a living cell counting device 1 according to the present embodiment includes a turbidity sensor 2, a first measuring instrument 3, a cell separation device 4, a second measuring instrument 5, and a calculation device 6. The turbidity sensor 2, the first measuring instrument 3, and the second measuring instrument 5 are connected to the calculation device 6 via a wired or wireless signal line.

In FIG. 1, the living cell counting device 1 is provided in a culture tank 7 in which cells are cultured and a useful substance is produced. A culture solution 8, which is a cell suspension, is put in the culture tank 7. The turbidity sensor 2 and the first measuring instrument 3 are provided in the culture tank 7. The cell separation device 4 is connected to the culture tank 7 via an extraction pipe 7a and a return pipe 7b. The return pipe 7b is provided with a circulation pump 9 through which the culture solution is circulated to the cell separation device 4. A discharge pipe 7c is connected to the cell separation device 4.

A living cell counting method used in the living cell counting device 1 includes: a step of measuring an amount of suspensoids contained in the cell suspension; a step of measuring capacitance of the cell suspension which varies depending on whether cells are living or dead; and a step of calculating one or more of the number of living cells, the number of dead cells, and a survival rate of cells contained in the cell suspension based on capacitance per unit of living cells obtained in advance, capacitance per unit of dead cells obtained in advance, the amount of the suspensoids, and the capacitance of the cell suspension.

The turbidity sensor 2 is provided to measure the amount of suspensoids contained in the cell suspension. The culture solution 8 in the culture tank 7 is a cell suspension containing cells as the suspensoids, and living cells, dead cells, and the like in a floating state are measured as the suspensoids in the culture solution 8. When the amount of the suspensoids is measured by the turbidity sensor 2, it is possible to obtain the total number of cells including living cells and dead cells contained in the culture solution 8, which is a cell suspension. In FIG. 1, the turbidity sensor 2 is provided in the culture tank 7 in an in-line manner, and a measuring unit is inserted into the culture tank 7.

As a method for measuring the amount of the suspensoids, various methods such as a transmitted light measurement method, a scattered light measurement method, a transmitted light and scattered light comparison method, an integrating sphere method, and a particle count method can be used. As the turbidity sensor 2, an absorption photometer, a laser and diffraction measuring device, or the like can be used. When such an optical system is used, the amount of the suspensoids contained in the cell suspension can be accurately measured at the same time as the measurement of the capacitance.

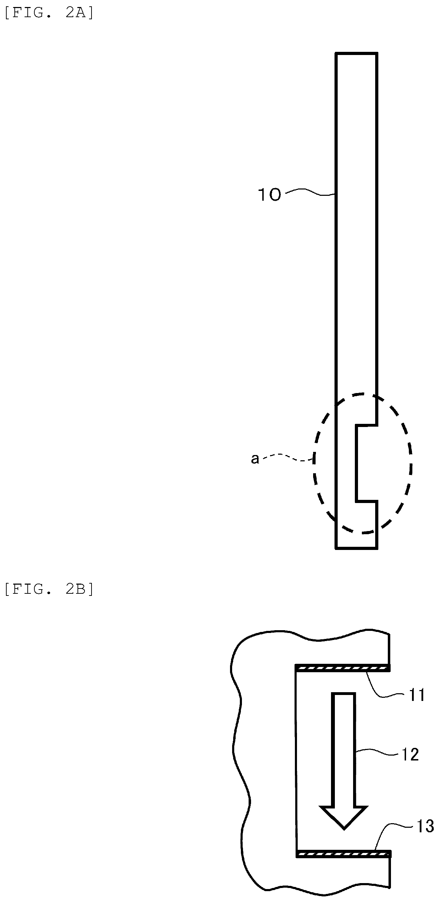

FIG. 2A is a diagram showing an example of a turbidity sensor according to the transmitted light measurement method. FIG. 2B is a partially enlarged view of FIG. 2A. FIG. 2B is the enlarged view of a portion surrounded by a broken line "a" in FIG. 2A.

As shown in FIG. 2A, as the turbidity sensor 2 based on the transmitted light measurement method, for example, a transmitted light type turbidity sensor 10 integrally provided with a light source can be used. The transmitted light type turbidity sensor 10 has a probe-shaped main body. A side surface on a distal end side of the main body is recessed from a main body side toward a central axis side. A recessed portion recessed toward the central axis side is a detection unit filled with a liquid to be measured.

In the transmitted light type turbidity sensor 10, at the time of measuring the amount of the suspensoids, the distal end side provided with the detection unit is immersed in the cell suspension to be measured. As shown in FIG. 2B, a light source 11 that emits light such as visible light or infrared light is provided on a surface of a base end side of the main body facing the detection unit. Meanwhile, a light receiving unit 13 that detects transmitted light 12 is provided on a surface on the distal end side of the main body. When measurement light is emitted from the light source 11, the transmitted light 12 transmitted through the cell suspension in the measuring unit reaches the light receiving unit 13, and intensity of the transmitted light 12 is measured.

The cell suspension includes the living cells, the dead cells, and the like that are suspended, as the suspensoids for absorbing and scattering light. The amount of the suspensoids contained in the cell suspension can be considered to be equivalent to the total number of cells contained in the cell suspension when an influence of a background of substances other than cells, for example, an influence of a suspensoid or a medium component smaller than the cells is sufficiently small.

Therefore, when turbidity (E) of the cell suspension is measured, a total number of cells (N_{T}) contained in the cell suspension can be obtained by the following Equation (1). However, in Equation (1), I₀ represents intensity of incident light, I represents intensity of transmitted light, k represents a constant according to a measurement condition, and N_{T} represents the total number of cells (cell concentration).
[Math. 1] $E = log \frac{{I}_{0}}{I} = K ⋅ {N}_{T}$ (1)

A relation represented by Equation (1) is established when a concentration of the suspensoid contained in the cell suspension is low. Therefore, at the time of obtaining the total number of cells (N_{T}) contained in the cell suspension, it is preferable to prepare a calibration curve using a cell suspension in which the total number of cells is known. The calibration curve is preferably prepared in a range in which the amount of suspensoids and the total number of cells are in a linear proportional relation, but the range is not limited to such a range.

For example, the total number of cells (N_{T}) contained in the cell suspension may also be obtained as a function having the amount of suspensoids (turbidity E) as a variable, as represented by the following Equation (2). The function having the amount of suspensoids as a variable is, for example, a linear function or the like, and can be obtained by measuring the amount of suspensoids with respect to the total number of cells using a plurality of cell suspensions in which the total number of cells is known and then fitting a function to a measurement result indicating a relation between the amount of suspensoids and the total number of cells that are contained in the cell suspension.
[Math. 2] ${N}_{T} = F \left(E\right)$ (2)

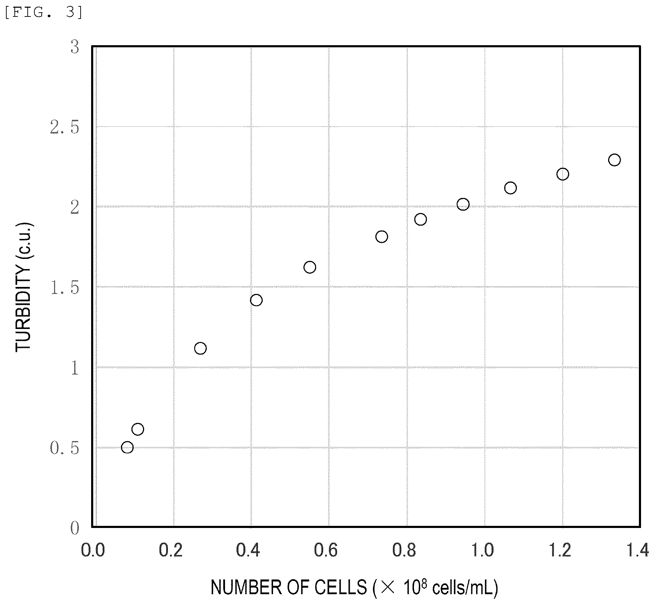

FIG. 3 is a diagram showing an example of a relation between turbidity of the cell suspension and the number of cells.

In FIG. 3, a vertical axis represents turbidity [a.u.] of the culture solution, and a horizontal axis represents the number of cells [× 10⁸ cells/mL] in the culture solution. FIG. 3 shows turbidity obtained when Chinese Hamster ovary (CHO) cells are cultured and results obtained by a related-art counting method using a hemocytometer.

As shown in FIG. 3, in a low concentration region, the turbidity of the culture solution and the total number of cells are in a linear proportional relation. Therefore, when the cell suspension to be measured for the number of living cells has a low concentration, a measurement value of the amount of suspensoids can be converted into the total number of cells by multiplying the measurement value of the amount of suspensoids by a constant. On the other hand, in a high concentration region, the turbidity of the culture solution and a total number of cells are in a nonlinear relation. Therefore, when the cell suspension has a high concentration, the measurement value of the amount of suspensoids is converted into the total number of cells using the function obtained by the fitting.

In FIG. 3, a relation between the turbidity of the culture solution and the total number of cells is obtained up to 1.4 × 10⁸ cells/mL. This cell concentration is at a level generally used in high-density perfusion culture. Therefore, from the results shown in FIG. 3, it can be said that, even when an animal cell or the like is cultured at a high density as in the perfusion culture, the total number of cells can be obtained from the amount of suspensoids as long as self-shielding by the suspensoids hardly occurs.

A measurement wavelength of the turbidity sensor 2 can be appropriately set according to the types of cells to be measured for the number of living cells and the like. In general, the sensitivity of measurement tends to improve as the measurement wavelength becomes shorter. The measurement wavelength of the turbidity sensor 2 is preferably in a range from a visible light region to a near-infrared light region from a viewpoint of increasing the sensitivity of measurement and non-invasively measuring the cells during culture.

The measurement wavelength of the turbidity sensor 2 is particularly preferably 600 ± 10 nm in the case of yeast, bacteria, or the like, 660 ± 10 nm in the case of an animal cell, an E. coli, or the like, and 730 ± 10 nm in the case of cyanobacteria, microalgae, or the like. From a viewpoint of reducing an influence of disturbance light and color, a near-infrared light region of 840 to 910 nm is preferable.

The first measuring instrument 3 is provided to measure the capacitance of the cell suspension which varies depending on whether cells are living or dead. As the first measuring instrument 3, for example, an impedance analyzer that measures impedance of a cell suspension, a multimeter, or a measuring instrument using a reflection transmission method, a lumped constant method, a resonance method, or the like can be used. In FIG. 1, the first measuring instrument 3 is provided in the culture tank 7 in an in-line manner, and the measuring unit is inserted into the culture tank 7.

A cell suspension such as the culture solution 8 in the culture tank 7 may contain both living cells and dead cells, and not only living cells but also dead cells may be polarized. When background capacitance of substances other than cells is sufficiently small, the capacitance of the cell suspension measured by the first measuring instrument 3 can be considered to be equivalent to the capacitance of total cells, which is a combination of the capacitance of total living cells and the capacitance of total dead cells.

Therefore, when the capacitance of the cell suspension is measured by the first measuring instrument 3, the number of living cells, the number of dead cells, and the survival rate of cells that are contained in the cell suspension can be calculated from simultaneous model equations of the capacitance per unit of living cells, the capacitance per unit of dead cells, the total number of cells contained in the cell suspension, and the capacitance of the cell suspension.

The cell separation device 4 is a device that separates cells contained in the cell suspension. The culture solution 8 in the culture tank 7 is drawn out to the cell separation device 4 during culture. In the cell separation device 4, the culture solution 8 drawn out from the culture tank 7 is separated into a concentrated culture solution containing cells and a cell-free solution containing a suspensoid or a medium component smaller than a cell. When a cell separation process is performed by the cell separation device 4, the concentrated culture solution containing cells is returned from the cell separation device 4 to the culture tank 7 through the return pipe 7b. The cell-free solution is discharged from the cell separation device 4 to an outside of the living cell counting device 1 through the discharge pipe 7c.

The cell separation process performed by the cell separation device 4 may be performed continuously or intermittently during the cell culture. However, when the number of living cells is monitored in real time with high accuracy or when the cell separation process is required for the perfusion culture, it is preferable to continuously perform the process.

As a method for separating cells, a centrifugal separation method, a filtration method using a hollow fiber membrane, a filtration method using a flat membrane, a rotary filter method, a gravitational settling method, or the like can be used. As the cell separation device 4, a centrifuge, a membrane separation device, a rotary filter type filtration device, a settling separation tank, or the like can be used. When such a method is used, since mechanical damage to cells during culture is small, the cells can be separated relatively non-invasively. Since the generation of dead cells can be reduced, efficient production and appropriate quality can be maintained when a useful substance is produced.

The second measuring instrument 5 is provided to measure capacitance of a cell-free solution from which the cells are separated. As the second measuring instrument 5, a measuring instrument similar to the first measuring instrument 3 can be used. In FIG. 1, the second measuring instrument 5 is provided in an in-line manner in the discharge pipe 7c connected to the cell separation device 4, and a measuring unit is inserted into the discharge pipe 7c.

The cell suspension such as the culture solution 8 in the culture tank 7 contains a suspensoid, a medium component, and the like smaller than the cell, and these components may also be polarized. When the background capacitance of substances other than such cells is large, the capacitance of the cell suspension measured by the first measuring instrument 3 cannot be considered to be equivalent to the capacitance of total cells, and thus the number of living cells cannot be calculated with high accuracy.

In such a case, when capacitance of a cell-free solution separated from the cell suspension is measured by the second measuring instrument 5, the background capacitance of substances other than cells can be excluded from the capacitance of the cell suspension. Since the capacitance of the cell suspension excluding the background capacitance of substances other than cells is considered to be equivalent to the capacitance of total cells, even when the cell suspension contains a suspensoid, a medium component, or the like smaller than the cell, the number of living cells, the number of dead cells, and the survival rate of the cells that are contained in the cell suspension can be obtained with high accuracy.

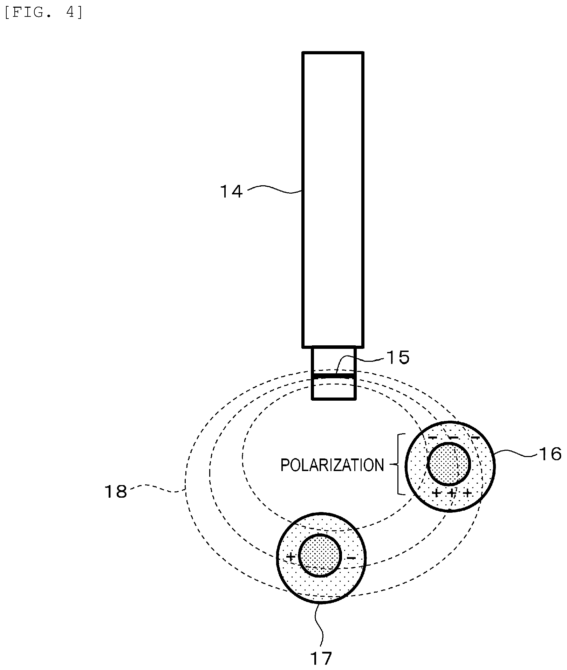

FIG. 4 is a diagram showing an example of a measuring instrument using the reflection transmission method.

As shown in FIG. 4, as the first measuring instrument 3 or the second measuring instrument 5, for example, a probe-type dielectric constant sensor 14 having an electrode at a distal end and using the reflection transmission method can be used. The dielectric constant sensor 14 includes a rod-shaped main body. An electrode probe 15 that is made of platinum or the like and that causes electromagnetic waves to be incident on a measurement target is provided at the distal end of the main body.

When the capacitance is measured, a distal end side of the dielectric constant sensor 14 on which the electrode probe 15 is provided is immersed in the cell suspension to be measured. As shown in FIG. 4, the cell suspension to be measured includes a living cell 16 and a dead cell 17. When an electric field 18 is generated by the electromagnetic waves from the electrode probe 15, the living cell 16 and the dead cell 17 generate polarization of different magnitudes.

Since the living cell 16 has a sound cell membrane as a dielectric, the living cell 16 is strongly polarized and has large capacitance. On the other hand, the dead cell 17 is less polarized than the living cell 16 or not polarized due to a damaged cell membrane. Therefore, when a reflected wave from the living cell 16 or the dead cell 17 is detected, the capacitance of total cells, which is a combination of the capacitance of total living cells and the capacitance of total dead cells, can be obtained from a reflection transmission characteristic of the reflected wave.

Each polarized cell in the cell suspension can be considered to be a capacitor. Therefore, the capacitance of total cells contained in the cell suspension is a value obtained by combining the capacitance per cell with the total number of cells. That is, the capacitance of total cells is a combination of the capacitance of total living cells obtained by synthesizing the capacitance per unit of living cells by the number of living cells per unit and the capacitance of total dead cells obtained by synthesizing the capacitance per unit of dead cells by the number of dead cells per unit.

In general, capacitance (C_{d}) per unit of dead cells is sufficiently smaller than capacitance (Cₗ) per unit of living cells. Therefore, in a general cell counting method in the related art in which the number of cells is estimated from capacitance, impedance, or dielectric constant, usually, it is considered that the capacitance of total cells corresponds to the capacitance of total living cells. On the other hand, in the living cell counting method according to the present embodiment, in order to consider the capacitance of dead cells, not only the capacitance (Cₗ) per unit of living cells and the number of living cells (Nₗ) but also the capacitance (C_{d}) per unit of dead cells and the number of dead cells (N_{d}) are added as variables.

Capacitance (C_{T}) of the cell suspension can be represented by the following Equation (3) with respect to a background capacitance (C_{B})of substances other than cells, capacitance (C_{L}) of total living cells, and capacitance (C_{D}) of total dead cells. Equation (3) is a relational expression simplified as capacitance in which cells are arranged in series. It is desirable to formulate the relational expression according to a cell arrangement within a capacitance measurement region. The total number of cells (N_{T}) contained in the cell suspension is represented by the following Equation (4) using the number of living cells (Nₗ) and the number of dead cells (N_{d}).
[Math. 3] $\frac{1}{{C}_{T}} + \frac{1}{{C}_{B}} = \frac{1}{{C}_{L}} + \frac{1}{{C}_{D}}$ (3)
[Math. 4] ${N}_{T} = {N}_{l} + {N}_{d}$ (4)

The capacitance (C_{L}) of total living cells can be represented by the following Equation (5) with respect to the capacitance (Cₗ) per unit of living cells and the number of living cells (N₁). The capacitance (C_{D}) of the dead cells can be represented by the following Equation (6) with respect to the capacitance (C_{d}) per unit of dead cells and the number of dead cells (N_{d}).
[Math. 5] $\frac{1}{{C}_{L}} = \frac{{N}_{l}}{{C}_{l}}$ (5)
[Math. 6] $\frac{1}{{C}_{D}} = \frac{{N}_{d}}{{C}_{d}}$ (6)

Therefore, simultaneous model equations represented by the following Equations (7) and (8) are established from Equations (3) to (6). When the background capacitance of substances other than cells is sufficiently small, a term C_{B} can be omitted.
[Math. 7] ${N}_{l} = \frac{{N}_{T} ⋅ {C}_{d}^{- 1} - \left({C}_{T}^{- 1}-{C}_{B}^{- 1}\right)}{{C}_{d}^{- 1} - {C}_{l}^{- 1}}$ (7)
[Math. 8] ${N}_{d} = \frac{- {N}_{T} ⋅ {C}_{l}^{- 1} + \left({C}_{T}^{- 1}-{C}_{B}^{- 1}\right)}{{C}_{d}^{- 1} - {C}_{l}^{- 1}}$ (8)

A survival rate (V) of the cells is represented by the following Equation (9) using Equation (2).
[Math. 9] $V = 1 - \frac{1 - {N}_{l}}{F \left(E\right)}$ (9)

By preparing a cell suspension in which the number of living cells is known and measuring capacitance of total cells contained in the cell suspension, the capacitance (Cₗ) per unit of living cells can be determined based on the capacitance of total cells and the known number of living cells. As the cell suspension in which the number of living cells is known, a cell suspension containing no dead cells is preferable, and, for example, a suspension or the like of cells in a growth period can be used.

By preparing a cell suspension in which the number of dead cells is known and measuring capacitance of total cells contained in the cell suspension, the capacitance (C_{d}) per unit of dead cells can be determined based on the capacitance of total cells and the known number of dead cells. As the cell suspension in which the number of dead cells is known, a cell suspension containing no living cells is preferable, and, for example, a suspension or the like subjected to a treatment for inducing cell death on cells in a growth period can be used. Examples of the treatment for inducing cell death include a treatment for causing mechanical damage and a treatment that depletes a nutrient.

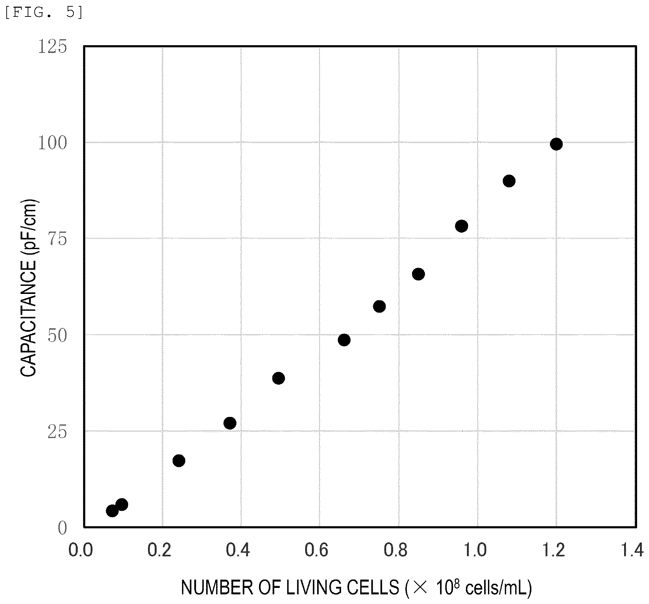

FIG. 5 shows an example of a relation between capacitance of the cell suspension and the number of living cells.

In FIG. 5, a vertical axis represents capacitance [pF/cm] of the cell suspension, and a horizontal axis represents the number of cells [× 10⁸ cells/mL] in the culture solution. FIG. 5 shows capacitance obtained when CHO cells are cultured and results obtained by the related-art counting method using the hemocytometer. As the cell suspension to be measured, a sample confirmed to have a cell survival rate of 95% or more is used.

In FIG. 5, a relation between the capacitance of the cell suspension and the number of living cells is substantially linear and is obtained up to 1.2 × 10⁸ cells/mL. This cell concentration is at a level generally used in high-density perfusion culture. Therefore, from the results shown in FIG. 5, it is understood that even when animal cells or the like are cultured at a high density as in perfusion culture, a correlation between the capacitance of the cell suspension and the number of living cells can be utilized by subtracting the background capacitance as necessary.

The calculation device 6 is implemented by a computer or the like including a calculation unit that calculates the simultaneous model equations represented by Equations (7) and (8), a storage unit that stores data of capacitance per unit of living cells obtained in advance and data of capacitance per unit of dead cells obtained in advance, an input unit that receives an input from an operator of the living cell counting device 1, a display unit that displays a calculation result, and the like.

The calculation unit of the calculation device 6 can be implemented by a central processing unit (CPU), a micro processing unit (MPU), or the like. The storage unit can be implemented by a storage device such as a hard disk drive (HDD) or a solid-state drive (SSD). The input unit can include input devices such as a keyboard, a mouse, and a touch panel. The display unit can be implemented by various display devices such as a liquid crystal display, a plasma display, an organic EL display, and a cathode-ray tube.

Data of the capacitance (Cₗ) per unit of living cells and data of the capacitance (C_{d}) per unit of dead cells are obtained in advance using a cell suspension in which the number of living cells and the number of dead cells are known, and then stored in the storage unit of the calculation device 6. As data per unit of cells, data of one cell may be prepared, or data of a predetermined cell population may be prepared. Data of a calibration curve indicating a relation between the amount of the suspensoids contained in the cell suspension and the total number of cells is obtained in advance using a cell suspension in which the number of cells is known, and then stored in the storage unit of the calculation device 6.

A signal of a measurement result of the amount of the suspensoids measured for the cell suspension to be measured is input from the turbidity sensor 2 to the calculation device 6 at predetermined time intervals. The data of the amount of the suspensoids is converted into data of the total number of cells based on the relation obtained in advance between the amount of the suspensoids and the total number of cells, and is used for calculation according to the simultaneous model equations.

A signal of a measurement result obtained by measuring the cell suspension to be measured is input to the calculation device 6 from the first measuring instrument 3 at predetermined time intervals. A signal of a measurement result obtained by measuring the cell-free solution from which the cells are separated is input from the second measuring instrument 5 at predetermined time intervals. Data such as impedance and transmission parameters measured by the first measuring instrument 3 and the second measuring instrument 5 are converted into data of the capacitance of the cell suspension and used for the calculation according to the simultaneous model equations.

The calculation device 6 calculates the simultaneous model equations represented by Equations (7) and (8) based on inputs of measurement results from the turbidity sensor 2, the first measuring instrument 3, and the second measuring instrument 5, and the data per unit of cells stored in the storage unit. That is, one or more of the number of living cells, the number of dead cells, and the survival rate of cells that are contained in the cell suspension are calculated based on the capacitance per unit of living cells obtained in advance, the capacitance per unit of dead cells obtained in advance, the amount of the suspensoids contained in the cell suspension, and the measured capacitance of the cell suspension.

The capacitance of the cell suspension measured for the cell suspension to be measured may be used for the calculation according to the simultaneous model equations without removing the background capacitance of substances other than cells, or may be used for the calculation according to the simultaneous model equations after removing the background capacitance of substances other than cells. When the background capacitance of substances other than cells is sufficiently small or when high accuracy is not required, a background process can be omitted.

According to the calculation according to the simultaneous model equations represented by Equations (7) and (8), since the number of living cells (Nₗ) and the number of dead cells (N_{d}) are unknown numbers among total number of cells (N_{T}), the number of living cells (Nₗ), the number of dead cells (N_{d}), the capacitance (C_{L}) of total living cells, the capacitance (Cₗ) per unit of living cells, and the capacitance (C_{d}) per unit of dead cells, the number of living cells (Nₗ) and the number of dead cells (N_{d}) can be obtained by simultaneously solving the two equations. The survival rate (V) of cells can be calculated from these results.

Results of the number of living cells (Nₗ), the number of dead cells (N_{d}), and the survival rate (V) of cells obtained by the calculation of the simultaneous model equations can be displayed on the display unit of the calculation device 6. These results may be displayed as time-series data, graphs, or the like along a measurement time of the amount of suspensoids contained in the cell suspension or the capacitance of the cell suspension. In addition to these results, data of the capacitance (Cₗ) per unit of living cells or data of the capacitance (C_{d}) per unit of dead cells may be displayed.

The living cell counting device 1 can measure one or more of the number of living cells (Nₗ), the number of dead cells (N_{d}), and the survival rate (V) of cells in real time or according to a predetermined schedule while cells to be measured are being cultured. The living cell counting device 1 may be used for a culture solution of any culture method such as batch culture, fed-batch culture, chemostat culture, or perfusion culture, in addition to a cell suspension prepared in advance.

### <Batch Culture>

The batch culture (batch culture) is a culture method in which a medium is prepared for each culture and no medium is supplied during the culture. According to the batch culture, when a useful substance is produced by the culture, the quality tends to vary for each culture, but there is an advantage that the risk of contamination can be dispersed or reduced.

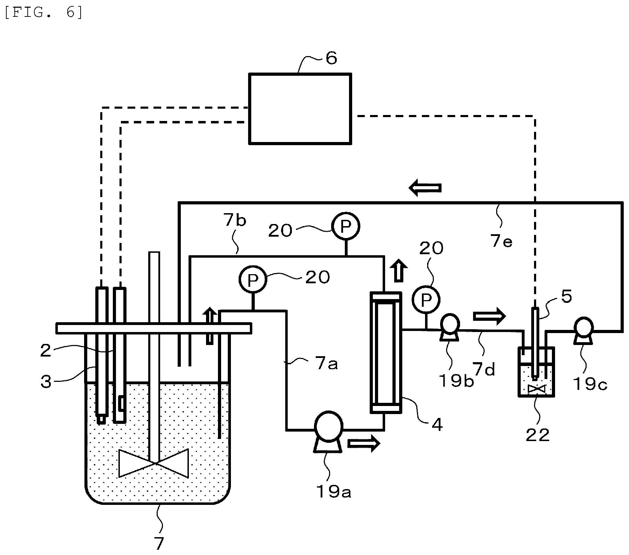

FIG. 6 is a diagram showing a batch culture apparatus including the living cell counting device.

As shown in FIG. 6, the living cell counting device can be provided in the batch culture apparatus. The batch culture apparatus includes the turbidity sensor 2, the first measuring instrument 3, the cell separation device 4, the second measuring instrument 5, and the calculation device 6, which constitute the living cell counting device. The batch culture apparatus further includes a circulation pump 19a, a transfer pump 19b, a return pump 19c, and a measurement tank 22.

In FIG. 6, the living cell counting device is provided in the culture tank 7 in which a useful substance is produced by batch culture. The turbidity sensor 2 and the first measuring instrument 3 are inserted into the culture tank 7. The cell separation device 4 is connected to the culture tank 7 via the extraction pipe 7a and the return pipe 7b. The extraction pipe 7a is provided with the circulation pump 19a through which the culture solution is circulated.

A measurement tank 22 is connected to the cell separation device 4 via a transfer pipe 7d. The second measuring instrument 5 is inserted into the measurement tank 22. The transfer pipe 7d is provided with the transfer pump 19b through which the cell-free solution is sent from the cell separation device 4 to the measurement tank 22. The measurement tank 22 is connected to the culture tank 7 via a return pipe 7e. The return pipe 7e is provided with the return pump 19c through which the cell-free solution is returned from the measurement tank 22 to the culture tank 7.

In order to control a culture environment, a batch culture tank 7 can include a pH sensor, a dissolved oxygen sensor, or a temperature sensor such as a thermocouple, a stirring device that stirs a culture solution, a ventilation device that passes air, oxygen, nitrogen, carbon dioxide, or the like through the culture solution, a heater that adjusts a temperature of the culture solution, a supply device that supplies an alkaline solution to the culture tank 7, and the like.

When floating cells are cultured, a stirrer provided with a stirring blade driven by a motor can be used. A culture atmosphere can be controlled by ventilation through a liquid surface, ventilation in the liquid, or both. The temperature of the culture solution is usually adjusted to an optimum temperature for culture or substance production by on or off control of the heater. The pH and the dissolved oxygen concentration are maintained at set values by feedback control or the like.

In the batch culture apparatus, culture of cells and substance production by the cells are performed in the culture solution put in the culture tank 7 without supplying a medium during the culture. In the batch culture apparatus, the capacitance (Cₗ) per unit of living cells and the capacitance (C_{d}) per unit of dead cells are obtained in advance before the culture, and data thereof is stored in the storage unit of the calculation device 6.

During batch culture of cells, the amount of suspensoids contained in the culture solution is measured over time by the turbidity sensor 2. The data of the measured amount of suspensoids is converted into data of the total number of cells (N_{T}) based on a relation between the amount of suspensoids (turbidity) and the number of cells that are contained in the culture solution.

During the batch culture of the cells, the capacitance (C_{T}) of the culture solution which is a cell suspension is measured over time by the first measuring instrument 3. When the background capacitance of substances other than cells is not sufficiently small, the capacitance of the cell-free solution separated from the culture solution is measured over time by the second measuring instrument 5, and the background capacitance of substances other than cells is removed from the capacitance of the culture solution.

The calculation device 6 calculates the number of living cells (Nₗ), the number of dead cells (N_{d}), and the survival rate (V) of cells from the simultaneous model equations of the total number of cells (N_{T}) of the culture solution, the capacitance (C_{T}) of the culture solution, the capacitance (Cₗ) per unit of living cells, and the capacitance (C_{d}) per unit of dead cells, using the data of the total number of cells (N_{T}) contained in the culture solution at the time of predetermined measurement and data of the capacitance (C_{T}) of the culture solution as inputs.

According to the batch culture apparatus including the living cell counting device, the number of living cells can be measured with high accuracy in real time during the batch culture in which dead cells are easily accumulated. During the batch culture, since consumption of a nutrient and accumulation of metabolites progress, the background capacitance of substances other than cells increases. However, when the background capacitance of substances other than cells is removed from the capacitance of the culture solution, higher accuracy can be obtained.

### <Fed-batch Culture>

The fed-batch culture (fed-batch culture) is a culture method in which a medium itself or a specific medium component is added from the outside of the system during culture, but the culture solution is not discharged until the culture is completed. According to the fed-batch culture, since the replenishment of a nutrient and the dilution of a metabolite are performed during the culture, the culture can be performed at a higher density than the batch culture, and a medium cost and the like can be reduced as compared with the perfusion culture.

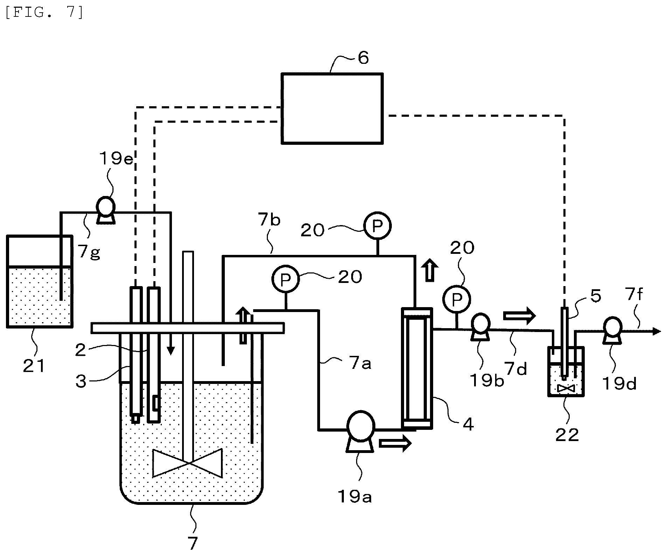

FIG. 7 is a diagram showing a fed-batch culture apparatus including the living cell counting device.

As shown in FIG. 7, the living cell counting device can be provided in the fed-batch culture apparatus. The fed-batch culture apparatus includes the turbidity sensor 2, the first measuring instrument 3, the cell separation device 4, the second measuring instrument 5, and the calculation device 6, which constitute the living cell counting device. The fed-batch culture apparatus further includes the circulation pump 19a, the transfer pump 19b, a discharge pump 19d, a medium supply pump 19e, a medium tank 21, and the measurement tank 22.

In FIG. 7, the living cell counting device is provided in the culture tank 7 in which a useful substance is produced by the fed-batch culture. The turbidity sensor 2 and the first measuring instrument 3 are inserted into the culture tank 7. The cell separation device 4 is connected to the culture tank 7 via the extraction pipe 7a and the return pipe 7b. The extraction pipe 7a is provided with the circulation pump 19a through which the culture solution is circulated.

The measurement tank 22 is connected to the cell separation device 4 via the transfer pipe 7d. The second measuring instrument 5 is inserted into the measurement tank 22. The transfer pipe 7d is provided with the transfer pump 19b through which the cell-free solution is sent from the cell separation device 4 to the measurement tank 22. A discharge pipe 7f is connected to the measurement tank 22. The discharge pipe 7f is provided with the discharge pump 19d through which the cell-free solution is discharged from the measurement tank 22 to the outside of the apparatus.

The medium tank 21 is connected to the culture tank 7 via a medium supply pipe 7g. A fresh medium is prepared in the medium tank 21. The medium supply pipe 7g is provided with the medium supply pump 19e through which the fresh medium is sent from the medium tank 21 to the culture tank 7.

Similar to the batch culture tank 7, in order to control the culture environment, a fed-batch culture tank 7 can include a pH sensor, a dissolved oxygen sensor, or a temperature sensor such as a thermocouple, a stirring device that stirs a culture solution, a ventilation device that passes air, oxygen, nitrogen, carbon dioxide, or the like through the culture solution, a heater that adjusts a temperature of the culture solution, a supply device that supplies an alkaline solution to the culture tank 7, and the like.

In the fed-batch culture apparatus, culture of cells and substance production by the cells are performed in the culture solution put in the culture tank 7 without supplying a fresh medium during the culture. Similar to the batch culture apparatus, in the fed-batch culture apparatus, the capacitance (Cₗ) per unit of living cells and the capacitance (C_{d}) per unit of dead cells are obtained in advance before the culture, and data thereof is stored in the storage unit of the calculation device 6.

During fed-batch culture of cells, the amount of suspensoids contained in the culture solution is measured over time by the turbidity sensor 2. The data of the measured amount of suspensoids is converted into the data of the total number of cells (N_{T}) based on the relation between the amount of suspensoids (turbidity) and the number of cells that are contained in the culture solution.

During the fed-batch culture of the cells, the capacitance (C_{T}) of the culture solution which is a cell suspension is measured over time by the first measuring instrument 3. When the background capacitance of substances other than cells is not sufficiently small, capacitance of a cell-free solution separated from the culture solution is measured over time by the second measuring instrument 5, and the background capacitance of substances other than cells is removed from the capacitance of the culture solution.

The calculation device 6 calculates the number of living cells (Nₗ), the number of dead cells (N_{d}), and the survival rate (V) of cells from simultaneous model equations of the total number of cells (N_{T}) of the culture solution, the capacitance (C_{T}) of the culture solution, the capacitance (Cₗ) per unit of living cells, and the capacitance (C_{d}) per unit of dead cells, using the data of the total number of cells (N_{T}) contained in the culture solution at the time of predetermined measurement and data of the capacitance (C_{T}) of the culture solution as inputs.

According to the fed-batch culture apparatus including the living cell counting device, the number of living cells can be measured with high accuracy in real time during the fed-batch culture in which dead cells are easily accumulated. During the fed-batch culture, since consumption of a nutrient and accumulation of metabolites progress, the background capacitance of substances other than cells increases. However, when the background capacitance of substances other than cells is removed from the capacitance of the culture solution, higher accuracy can be obtained.

### <Chemostat Culture>

The chemostat culture is a culture method in which a medium is continuously supplied during culture, and the same amount of culture solution containing cells is continuously discharged. According to the chemostat culture, since the culture environment is kept substantially constant, the productivity of a substance can be stabilized.

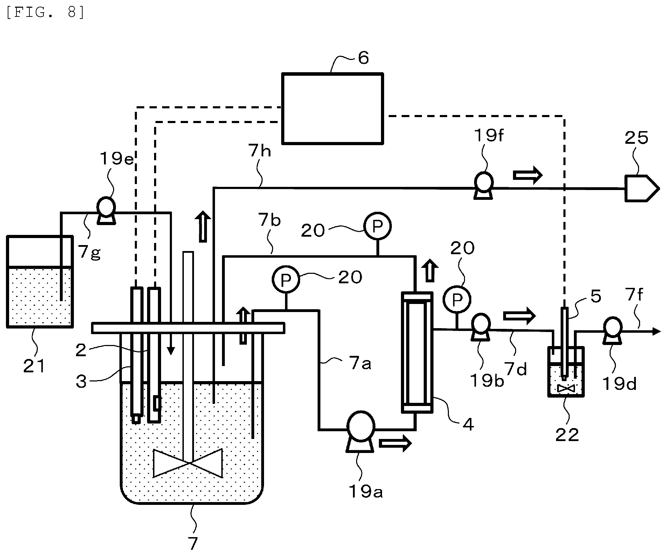

FIG. 8 is a diagram showing a chemostat culture apparatus including the living cell counting device.

As shown in FIG. 8, the living cell counting device can be provided in the chemostat culture apparatus. The chemostat culture apparatus includes the turbidity sensor 2, the first measuring instrument 3, the cell separation device 4, the second measuring instrument 5, and the calculation device 6, which constitute the living cell counting device. The chemostat culture apparatus further includes the circulation pump 19a, the transfer pump 19b, the discharge pump 19d, the medium supply pump 19e, a culture solution extraction pump 19f, the medium tank 21, and the measurement tank 22.

In FIG. 8, the living cell counting device is provided in the culture tank 7 in which a useful substance is produced by chemostat culture. The turbidity sensor 2 and the first measuring instrument 3 are inserted into the culture tank 7. The cell separation device 4 is connected to the culture tank 7 via the extraction pipe 7a and the return pipe 7b. The extraction pipe 7a is provided with the circulation pump 19a through which the culture solution is circulated.

The measurement tank 22 is connected to the cell separation device 4 via the transfer pipe 7d. The second measuring instrument 5 is inserted into the measurement tank 22. The transfer pipe 7d is provided with the transfer pump 19b through which the cell-free solution is sent from the cell separation device 4 to the measurement tank 22. The discharge pipe 7f is connected to the measurement tank 22. The discharge pipe 7f is provided with the discharge pump 19d through which the cell-free solution is discharged from the measurement tank 22 to the outside of the apparatus.

The medium tank 21 is connected to the culture tank 7 via the medium supply pipe 7g. A fresh medium is prepared in the medium tank 21. The medium supply pipe 7g is provided with the medium supply pump 19e through which the fresh medium is sent from the medium tank 21 to the culture tank 7. An extraction pipe 7h is connected to the culture tank 7. The extraction pipe 7h is provided with the culture solution extraction pump 19f through which a part of the culture solution containing cells is extracted from the culture tank 7. The culture solution extracted from the culture tank 7 is sent to the recovery system 25 together with the useful substance produced by the cells.

Similar to the batch culture tank 7, in order to control the culture environment, a chemostat culture tank 7 can include a pH sensor, a dissolved oxygen sensor, or a temperature sensor such as a thermocouple, a stirring device that stirs a culture solution, a ventilation device that passes air, oxygen, nitrogen, carbon dioxide, or the like through the culture solution, a heater that adjusts a temperature of the culture solution, a supply device that supplies an alkaline solution to the culture tank 7, and the like.

In the chemostat culture apparatus, a fresh medium is supplied to a culture solution put in the culture tank 7 during the culture, and culture of cells and substance production by the cells are performed while extracting a part of a culture solution containing grown cells. A supply rate of the fresh medium and an extraction rate of the culture solution are kept constant, and the temperature, pH, and the dissolved oxygen concentration in the culture tank 7 are kept constant. Similar to the batch culture apparatus, in the chemostat culture apparatus, the capacitance (Cₗ) per unit of living cells and the capacitance (C_{d}) per unit of dead cells are obtained in advance before the culture, and data thereof is stored in the storage unit of the calculation device 6.

During chemostat culture of cells, the amount of suspensoids contained in the culture solution is measured over time by the turbidity sensor 2. The data of the measured amount of suspensoids is converted into the data of the total number of cells (N_{T}) based on the relation between the amount of suspensoids (turbidity) and the number of cells that are contained in the culture solution.

During the chemostat culture of the cells, the capacitance (C_{T}) of the culture solution which is a cell suspension is measured over time by the first measuring instrument 3. When the background capacitance of substances other than cells is not sufficiently small, capacitance of a cell-free solution separated from the culture solution is measured over time by the second measuring instrument 5, and the background capacitance of substances other than cells is removed from the capacitance of the culture solution.

The calculation device 6 calculates the number of living cells (Nₗ), the number of dead cells (N_{d}), and the survival rate (V) of cells from simultaneous model equations of the total number of cells (N_{T}) of the culture solution, the capacitance (C_{T}) of the culture solution, the capacitance (Cₗ) per unit of living cells, and the capacitance (C_{d}) per unit of dead cells, using the data of the total number of cells (N_{T}) contained in the culture solution at the time of predetermined measurement and data of the capacitance (C_{T}) of the culture solution as inputs.

According to the chemostat culture apparatus including the living cell counting device, the number of living cells can be measured with high accuracy in real time during the chemostat culture in which cells are sequentially extracted. During the chemostat culture, the number of cells in the culture tank may vary due to extraction of cells. However, since the living cell counting device can measure the number of living cells with high accuracy in real time, a supply amount of the fresh medium and an extraction amount of the culture solution can be properly adjusted.

### <Perfusion Culture>

The perfusion culture is a culture method in which a medium is continuously supplied during culture, and the same amount of culture solution containing cells is continuously discharged. According to the perfusion culture, since the culture environment is easily kept constant, the productivity of a substance can be stabilized. Since the extraction of the grown cells is reduced, the cells can be cultured at a higher density than that in the chemostat culture.

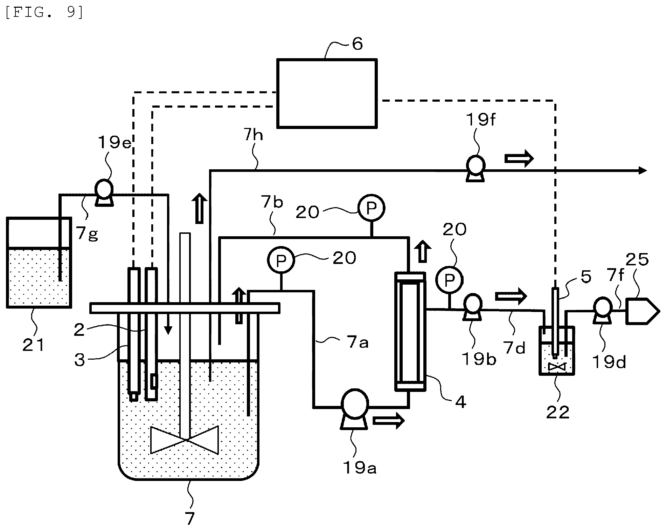

FIG. 9 is a diagram showing a perfusion culture apparatus including the living cell counting device.

As shown in FIG. 9, the living cell counting device can be provided in the perfusion culture apparatus. The perfusion culture apparatus includes the turbidity sensor 2, the first measuring instrument 3, the cell separation device 4, the second measuring instrument 5, and the calculation device 6, which constitute the living cell counting device. The perfusion culture apparatus further includes the circulation pump 19a, the transfer pump 19b, the discharge pump 19d, the medium supply pump 19e, the culture solution extraction pump 19f, the medium tank 21, and the measurement tank 22.

In FIG. 9, the living cell counting device is provided in the culture tank 7 in which a useful substance is produced by perfusion culture. The turbidity sensor 2 and the first measuring instrument 3 are inserted into the culture tank 7. The cell separation device 4 is connected to the culture tank 7 via the extraction pipe 7a and the return pipe 7b. The extraction pipe 7a is provided with the circulation pump 19a through which the culture solution is circulated.

The measurement tank 22 is connected to the cell separation device 4 via the transfer pipe 7d. The second measuring instrument 5 is inserted into the measurement tank 22. The transfer pipe 7d is provided with the transfer pump 19b through which the cell-free solution is sent from the cell separation device 4 to the measurement tank 22. The discharge pipe 7f is connected to the measurement tank 22. The discharge pipe 7f is provided with the discharge pump 19d through which the cell-free solution is discharged from the measurement tank 22 to the outside of the apparatus. The cell-free solution extracted from the measurement tank 22 is sent to the recovery system 25 together with the useful substance produced by the cells.

The medium tank 21 is connected to the culture tank 7 via the medium supply pipe 7g. A fresh medium is prepared in the medium tank 21. The medium supply pipe 7g is provided with the medium supply pump 19e through which the fresh medium is sent from the medium tank 21 to the culture tank 7. The extraction pipe 7h is connected to the culture tank 7. The extraction pipe 7h is provided with the culture solution extraction pump 19f through which a part of the culture solution containing cells is extracted from the culture tank 7 to the outside of the device. The extraction pipe 7h is used to extract excessively cultured cells as bleeding.

Similar to the batch culture tank 7, in order to control the culture environment, a perfusion culture tank 7 can include a pH sensor, a dissolved oxygen sensor, or a temperature sensor such as a thermocouple, a stirring device that stirs a culture solution, a ventilation device that passes air, oxygen, nitrogen, carbon dioxide, or the like through the culture solution, a heater that adjusts a temperature of the culture solution, a supply device that supplies an alkaline solution to the culture tank 7, and the like.

In the perfusion culture apparatus, a fresh medium is supplied in a culture solution put in the culture tank 7 during the culture, and culture of cells and substance production by the cells are performed while extracting a cell-free solution from which the cells are separated. A supply rate of the fresh medium and an extraction rate of the cell-free solution are kept constant, and the temperature, pH, and the dissolved oxygen concentration in the culture tank 7 are kept constant. Similar to the batch culture apparatus, in the perfusion culture apparatus, the capacitance (Cₗ) per unit of living cells and the capacitance (C_{d}) per unit of dead cells are obtained in advance before the culture, and data thereof is stored in the storage unit of the calculation device 6.

During perfusion culture of cells, the amount of suspensoids contained in the culture solution is measured over time by the turbidity sensor 2. The data of the measured amount of suspensoids is converted into the data of the total number of cells (N_{T}) based on the relation between the amount of suspensoids (turbidity) and the number of cells that are contained in the culture solution.

During the perfusion culture of the cells, the capacitance (C_{T}) of the culture solution which is a cell suspension is measured over time by the first measuring instrument 3. When the background capacitance of substances other than cells is not sufficiently small, capacitance of a cell-free solution separated from the culture solution is measured over time by the second measuring instrument 5, and the background capacitance of substances other than cells is removed from the capacitance of the culture solution.

The calculation device 6 calculates the number of living cells (Nₗ), the number of dead cells (N_{d}), and the survival rate (V) of cells from simultaneous model equations of the total number of cells (N_{T}) of the culture solution, the capacitance (C_{T}) of the culture solution, the capacitance (Cₗ) per unit of living cells, and the capacitance (C_{d}) per unit of dead cells, using the data of the total number of cells (N_{T}) contained in the culture solution at the time of predetermined measurement and data of the capacitance (C_{T}) of the culture solution as inputs.

According to the perfusion culture apparatus including the living cell counting device, the number of living cells can be measured with high accuracy in real time during the perfusion culture in which cells are continuously cultured. In the related art, in the perfusion culture, a cell separation process is performed when a part of the culture solution is extracted. In the living cell counting device, it is possible to use an existing cell separation device used for such a cell separation process to collect a cell-free solution necessary for measuring the background capacitance of substances other than cells.

According to the above living cell counting method and the living cell counting device, the capacitance per unit of living cells and the capacitance per unit of dead cells are incorporated into the simultaneous model equations of the total number of cells contained in the cell suspension and the capacitance of the cell suspension, and the number of living cells, the number of dead cells, and the survival rate of cells are calculated. Therefore, even when dead cells have capacitance, such as cells are dead with cell membranes left, the number of living cells can be measured with high accuracy. Since the total number of cells contained in the cell suspension is measured by an optical method as the amount of suspensoids, a measurement result represented by a unit of the number of cells or the cell concentration is obtained.

The amount of suspensoids contained in the cell suspension and the capacitance of the cell suspension can be measured in real time by impedance measurement or turbidity measurement in a predetermined frequency range. Unlike the case of sampling cells, there is little risk of germs being mixed or the like during measurement, and there is no invasive influence on cells as in the case of staining or controlling the dissolved oxygen concentration. Thus, the number of living cells included in cells can be measured with high accuracy by non-invasive means with a low risk of contamination.

Although the living cell counting device 1 includes the cell separation device 4 and the second measuring instrument 5, the installation of the cell separation device 4 and the second measuring instrument 5 may be omitted when a background process of the capacitance of the cell suspension is not performed. As the turbidity sensor 2, the first measuring instrument 3, and the second measuring instrument 5, for example, a probe-type in-line sensor is provided, but other types of sensors may be used as long as sampling is not performed.

In the above living cell counting device 1 and the living cell counting method, the simultaneous model equations represented by Equations (7) and (8) are used for the calculation, but other simultaneous model equations may be used for the calculation in accordance with a spatial arrangement of the cells for which the capacitance is measured, a range of a background to be considered, and the like as long as the total number of cells (N_{T}), the number of living cells (Nₗ), the number of dead cells (N_{d}), the capacitance (C_{L}) of total living cell, the capacitance (Cₗ) per unit of living cells, and the capacitance (C_{d}) per unit of dead cells are variables.

### [Examples]

Next, the invention will be described in detail with reference to the examples, but the technical scope of the invention is not limited thereto.

### <Example 1>

In the batch culture, an amount of suspensoids contained in a cell suspension and capacitance of the cell suspension were measured, and the number of living cells was measured based on the simultaneous model equations represented by Equations (7) and (8).

### (Cell and Medium)

As cells to be measured for the number of living cells, CHO cells (ATCC CRL-12445 cells), which are genetically modified organisms that produce antibodies (IgG) and are ordered into floating cells, were used. As a medium, a medium produced by adding insulin (final concentration: 10 µg/mL), transferrin (final concentration: 10 µg/mL), and fetal bovine serum (FBS) (final concentration: 10 µg/mL) to a Dulbecco's modified eagle medium (DMEM) was used.

### (Experimental Device)

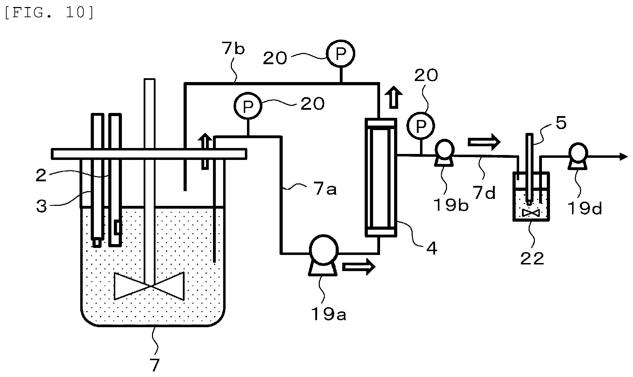

FIG. 10 is a diagram showing the living cell counting device used in Example 1 in which the batch culture is performed.

As shown in FIG. 10, an apparatus in which the turbidity sensor 2, the first measuring instrument 3 which is an electrostatic capacitance meter, the cell separation device 4 that performs filtration by a hollow fiber membrane, and the second measuring instrument 5 which is an electrostatic capacitance meter are attached to the batch culture tank 7 was used as the living cell counting device.

The CHO cells to be measured were cultured in the batch culture tank 7, and the number of living cells was measured in real time. For comparison, a measurement in the related art using a hemocytometer was performed in parallel. The measurement in the related art was performed by a method for sampling a culture solution once a day, staining dead cells with trypan blue, and counting cells visually observed in a microscope field of view using a hemocytometer.

### (Experimental Result)

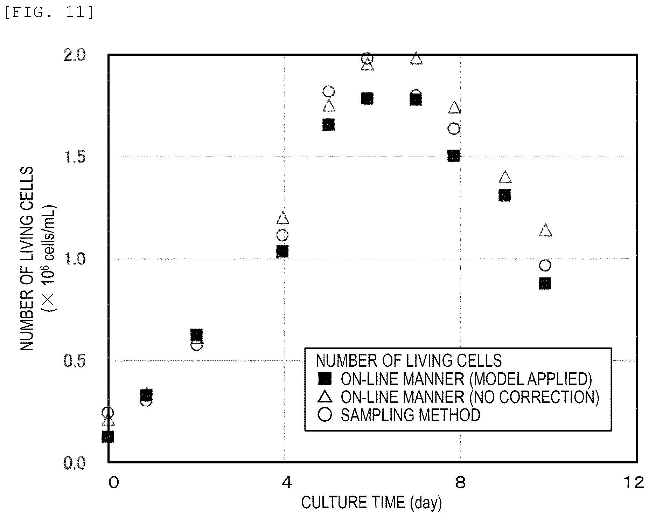

FIG. 11 is a diagram showing measurement results of the number of living cells in Example 1 in which the batch culture is performed.

In FIG. 11, the horizontal axis represents a culture time [day], and the vertical axis represents the measured number of living cells [× 10⁶ cells/mL]. A plot of o indicates a measurement result obtained by a related-art method in an off-line manner that performs sampling. A plot of Δ indicates a measurement result obtained by a related-art method in an in-line manner that estimates the number of cells from the measured capacitance. A plot of ■ indicates a measurement result according to Example 1 in which the measurement of the amount of suspensoids and the measurement of the capacitance of the cell suspension were performed and the calculation was performed based on the model equations.

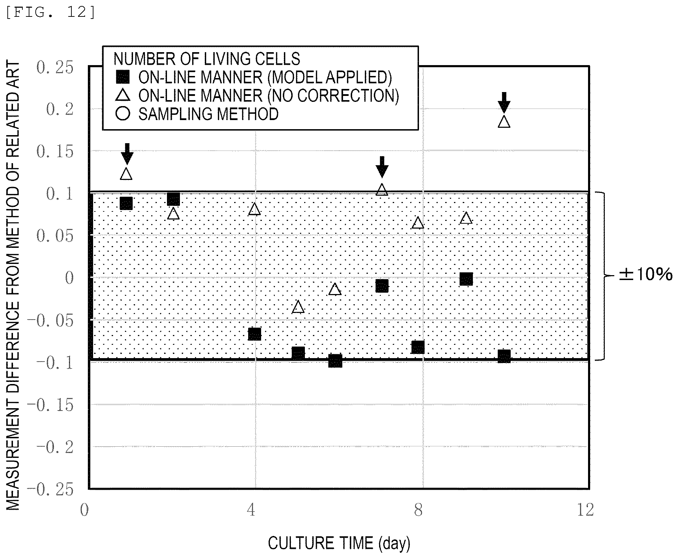

FIG. 12 is a diagram showing a deviation of the measurement results in Example 1 from those obtained by a method in the related art.

FIG. 12 shows a comparison between a measurement difference between the measurement results of the number of living cells according to Example 1 and the measurement results obtained by the related-art method in an off-line manner and a measurement difference between the measurement results obtained by the related-art method in an in-line manner that estimates the number of cells from the capacitance and the measurement results obtained by the related-art method in an off-line manner. Each plot in FIG. 12 corresponds to a plot in FIG. 11. Since dead cells are stained with trypan blue and counted by the hemocytometer, the measurement results obtained by the related-art method in an off-line manner are considered to be the measurement with the highest accuracy.

As shown in FIG. 12, the measurement results of the number of living cells according to Example 1 calculated based on the simultaneous model equations represented by Equations (7) and (8) are less likely to vary with respect to the count obtained using a hemocytometer, compared with the measurement results obtained by the related-art method in an in-line manner that estimates the number of cells from the measured capacitance. In the related-art method in an in-line manner, a variation with respect to the count obtained using the hemocytometer frequently occurs, and in particular, the variation increases in a late stage of culture in which the survival rate of cells decreases.

From these results, it can be seen that, according to a cell counting method according to Example 1 in which calculation is performed based on the simultaneous model equations represented by Equations (7) and (8), a number close to the count obtained using the hemocytometer in the related art can be obtained with high accuracy in the batch culture.

### <Example 2>

In the perfusion culture, an amount of suspensoids contained in a cell suspension and capacitance of the cell suspension were measured, and the number of living cells was measured based on the simultaneous model equations represented by Equations (7) and (8).

### (Cell and Medium)

As cells to be measured for the number of living cells, as in Example 1, CHO cells (ATCC CRL-12445 cells), which are genetically modified organisms that produce antibodies (IgG) and are ordered into floating cells, were used. As a medium, as in Example 1, a medium produced by adding insulin (final concentration: 10 µg/mL), transferrin (final concentration: 10 µg/mL), and fetal bovine serum (FBS) (final concentration: 10 µg/mL) to a Dulbecco's modified eagle medium (DMEM) was used.

### (Experimental Device)

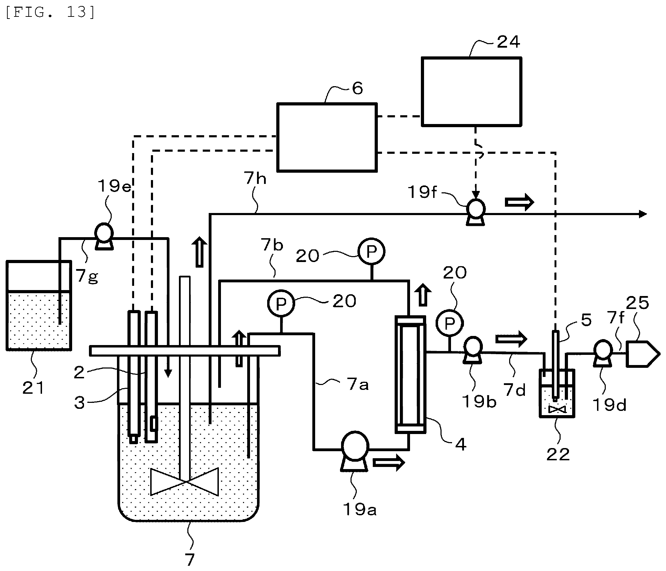

FIG. 13 is a diagram showing the living cell counting device used in Example 2 in which the perfusion culture is performed.

As shown in FIG. 13, an apparatus in which the turbidity sensor 2, the first measuring instrument 3 which is an electrostatic capacitance meter, the cell separation device 4 that performs filtration by a hollow fiber membrane, the second measuring instrument 5 which is an electrostatic capacitance meter, the calculation device 6, and a control device 24 are attached to the perfusion culture tank 7 was used as the living cell counting device. A volume of the culture tank 7 was 1 L. The cell separation device 4 was provided with a hollow fiber membrane having a cut-off molecular weight of 300 kDa.

The CHO cells to be measured were cultured in the perfusion culture tank 7, and the number of living cells was measured in real time. As seed cells, the CHO cells diluted to 1 × 10⁵ cells/mL in the DMEM medium were seeded in the culture tank 7. Culture conditions were maintained at a culture temperature of 37°C, a dissolved oxygen concentration of 2.7 mg/L, and a pH of 7.2. A circulation rate of the culture solution to the cell separation device 4 was 10 mL/min, and a perfusion rate was set at 1 vvd (30 mL/h) by supplying a fresh medium.

During the perfusion culture, the number of living cells was measured by the calculation device 6, and bleeding through the extraction pipe 7h was controlled based on a measurement result on the 21st day from the start of the culture. An amount of bleeding was adjusted by controlling an output of the culture solution extraction pump 19f by the control device 24 such that the number of living cells in the culture tank 7 was maintained at 1 × 10⁷ cells/mL.

### (Experimental Result)

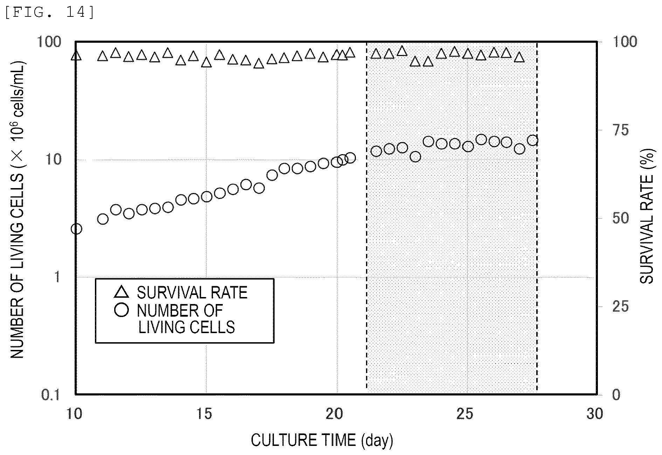

FIG. 14 is a diagram showing measurement results of the number of living cells in Example 2 in which the perfusion culture is performed.

In FIG. 14, a horizontal axis represents the culture time [day], and a vertical axis represents the measured number of living cells [× 10⁶ cells/mL] or a survival rate of cells [%]. A plot of o indicates the number of living cells. A plot of Δ indicates the survival rate of cells. A section between broken lines indicates a period in which the cell concentration is controlled.

As shown in FIG. 14, since the measurement results of the number of living cells according to Example 2 calculated based on the simultaneous model equations represented by Equations (7) and (8) were also obtained with high accuracy in the perfusion culture, the number of living cells could be kept constant even at a stage in which the culture time elapsed.

From these results, it can be seen that a cell counting method according to Example 2 in which calculation is performed based on the simultaneous model equations represented by Equations (7) and (8) can be used for feedback control or the like for keeping the number of living cells constant in the perfusion culture.

### Reference Signs List

1 living cell counting device
2 turbidity sensor
3 first measuring instrument
4 cell separation device
5 second measuring instrument
6 calculation device
7 culture tank
8 culture solution
10 transmitted light turbidity sensor
11 light source
12 transmitted light
13 light receiving unit
14 dielectric constant sensor
15 electrode probe
16 living cell
17 dead cell
19a circulation pump
19b transfer pump
19c return pump
19d discharge pump
19e medium supply pump
19f culture solution extraction pump
21 medium tank
22 measurement tank
24 control device
25 recovery system

## Claims

1. A living cell counting method comprising:
a step of measuring an amount of suspensoids contained in a cell suspension;
a step of measuring capacitance of the cell suspension which varies depending on whether cells are living or dead; and
a step of calculating one or more of the number of living cells, the number of dead cells, and a survival rate of cells that are contained in the cell suspension, based on the amount of the suspensoids, and the capacitance of the cell suspension,
**characterized in that** the step of calculating the one or more of the number of living cells, the number of dead cells, and a survival rate of cells that are contained in the cell suspension, is further based on capacitance per unit of living cells obtained in advance and capacitance per unit of dead cells obtained in advance.

2. The living cell counting method according to claim 1, further comprising:
a step of measuring capacitance of a cell suspension in which the number of living cells is known to obtain capacitance per unit of living cells; and
a step of measuring capacitance of a cell suspension in which the number of dead cells is known to obtain capacitance per unit of dead cells.

3. The living cell counting method according to claim 1, wherein
the capacitance of the cell suspension is used for calculation after background capacitance of substances other than cells is removed.

4. The living cell counting method according to claim 3, further comprising:
a step of separating cells contained in the cell suspension; and
a step of measuring capacitance of a cell-free solution from which the cells are separated.

5. A living cell counting device (1) comprising:
a turbidity sensor (2) configured to measure an amount of suspensoids contained in a cell suspension;
a measuring instrument (3) configured to measure capacitance of the cell suspension which varies depending on whether cells are living or dead; and
a calculation unit configured to calculate one or more of the number of living cells, the number of dead cells, and a survival rate of cells that are contained in the cell suspension, based on the amount of the suspensoids, and the capacitance of the cell suspension,
**characterized in that** the calculation of the one or more of the number of living cells, the number of dead cells, and a survival rate of cells that are contained in the cell suspension, is further based on the capacitance per unit of living cells and the capacitance per unit of dead cells.

6. The living cell counting device (1) according to claim 5, further comprising:
a storage unit configured to store data of the capacitance per unit of living cells and the capacitance per unit of dead cells.

7. The living cell counting device (1) according to claim 5, wherein
the capacitance of the cell suspension is used for calculation after background capacitance of substances other than cells is removed.

8. The living cell counting device (1) according to claim 7, further comprising:
a cell separation device (4) configured to separate cells contained in the cell suspension; and
a measuring instrument (5) configured to measure capacitance of a cell-free solution from which the cells are separated.

9. The living cell counting device (1) according to claim 8, wherein
examples of a method for separating the cells include a centrifugal separation method, a filtration method using a hollow fiber membrane, a filtration method using a flat membrane, a rotary filter method, and a gravitational settling method.

10. The living cell counting device (1) according to claim 5, wherein
examples of a method for measuring the amount of the suspensoids include a transmitted light measurement method, a scattered light measurement method, a transmitted light and scattered light comparison method, an integrating sphere method, or a particle count method.

## Patentansprüche

1. Zählverfahren für lebende Zellen, umfassend:
einen Schritt des Messens einer Menge von Suspensoiden, die in einer Zellsuspension enthalten sind;
einen Schritt des Messens der Kapazitanz der Zellsuspension, die abhängig davon variiert, ob die Zellen lebend oder tot sind; und
einen Schritt des Berechnens einer oder mehrerer aus der Anzahl lebender Zellen, der Anzahl toter Zellen und einer Überlebensrate von Zellen, die in der Zellsuspension enthalten sind, basierend auf der Menge der Suspensoide und der Kapazitanz der Zellsuspension,
**dadurch gekennzeichnet, dass** der Schritt des Berechnens der einen oder der mehreren aus der Anzahl lebender Zellen, der Anzahl toter Zellen und einer Überlebensrate von Zellen, die in der Zellsuspension enthalten sind, weiters auf der Kapazitanz pro Einheit lebender Zellen, die im Vorhinein erhalten wurde, und der Kapazitanz pro Einheit toter Zellen, die im Vorhinein erhalten wurde, basiert.

2. Zählverfahren für lebende Zellen nach Anspruch 1, das weiters Folgendes umfasst:
einen Schritt des Messens der Kapazitanz einer Zellsuspension, in der die Anzahl lebender Zellen bekannt ist, um eine Kapazitanz pro Einheit lebender Zellen zu erhalten; und
einen Schritt des Messens der Kapazitanz einer Zellsuspension, in der die Anzahl toter Zellen bekannt ist, um eine Kapazitanz pro Einheit toter Zellen zu erhalten.

3. Zählverfahren für lebende Zellen nach Anspruch 1, wobei:
die Kapazitanz der Zellsuspension zur Berechnung verwendet wird, nachdem die Hintergrundkapazitanz von Substanzen, die nicht die Zellen sind, entfernt wurde.

4. Zählverfahren für lebende Zellen nach Anspruch 3, das weiters Folgendes umfasst:
einen Schritt des Abtrennens von Zellen, die in der Zellsuspension enthalten sind; und
einen Schritt des Messens der Kapazitanz einer zellfreien Lösung, aus der die Zellen abgetrennt werden.

5. Zählvorrichtung (1) für lebende Zellen, umfassend:
einen Trübungssensor (2), der ausgelegt ist, um eine Menge an Suspensoiden zu messen, die in einer Zellsuspension enthalten sind;
ein Messinstrument (3), das ausgelegt ist, um die Kapazitanz der Zellsuspension zu messen, die abhängig davon variiert, ob die Zellen lebendig oder tot sind; und
eine Berechnungseinheit, die ausgelegt ist, um eine oder mehrere aus der Anzahl lebender Zellen, der Anzahl toter Zellen und einer Überlebensrate von Zellen, die in der Zellsuspension enthalten sind, basierend auf der Menge der Suspensoide und der Kapazitanz der Zellsuspension zu berechnen,
**dadurch gekennzeichnet, dass** das Berechnen der einen oder der mehreren aus der Anzahl lebender Zellen, der Anzahl toter Zellen und einer Überlebensrate von Zellen, die in der Zellsuspension enthalten sind, weiters auf der Kapazitanz pro Einheit lebender Zellen und der Kapazitanz pro Einheit toter Zellen basiert.

6. Zählvorrichtung (1) für lebende Zellen nach Anspruch 5, die weiters Folgendes umfasst:
eine Speichereinheit, die ausgelegt ist, um Daten der Kapazitanz pro Einheit lebender Zellen und der Kapazitanz pro Einheit toter Zellen zu speichern.

7. Zählvorrichtung (1) für lebende Zellen nach Anspruch 5, wobei:
die Kapazitanz der Zellsuspension zur Berechnung verwendet wird, nachdem die Hintergrundkapazitanz von Substanzen, die nicht die Zellen sind, entfernt wurde.

8. Zählvorrichtung (1) für lebende Zellen nach Anspruch 7, die weiters Folgendes umfasst:
eine Zellabtrennungsvorrichtung (4), die ausgelegt ist, um Zellen, die in der Zellsuspension enthalten sind, abzutrennen; und
ein Messinstrument (5), das ausgelegt ist, um die Kapazitanz einer zellfreien Lösung, von der die Zellen abgetrennt wurden, zu messen.

9. Zählvorrichtung (1) für lebende Zellen nach Anspruch 8, wobei:
Beispiele für ein Verfahren zum Abtrennen der Zellen ein Zentrifugalabtrennverfahren, ein Filtrationsverfahren unter Verwendung einer Hohlfasermembran, ein Filtrationsverfahren unter Verwendung einer flachen Membran, ein Rotationsfilterverfahren und ein Schwerkraftabsetzverfahren umfassen.

10. Zählvorrichtung (1) für lebende Zellen nach Anspruch 5, wobei:
Beispiele für ein Verfahren zum Messen der Menge der Suspensoide ein Durchlicht-Messverfahren, ein Messverfahren von gestreutem Licht, ein Vergleichsverfahren von Durchlicht und gestreutem Licht, ein Ulbricht-Kugel-Verfahren oder ein Teilchenzählverfahren umfassen.

## Revendications

1. Procédé de comptage de cellules vivantes, comprenant :
une étape de mesure de la quantité de matières en suspension contenue dans une suspension cellulaire ;
une étape de mesure de la capacité de la suspension cellulaire qui varie selon que les cellules soient vivantes ou mortes ; et
une étape de calcul d'un ou de plusieurs parmi le nombre de cellules vivantes, le nombre de cellules mortes, et un taux de survie des cellules qui sont contenues dans la suspension cellulaire, sur la base de la quantité des matières en suspension, et de la capacité de la suspension cellulaire,
**caractérisé en ce que** l'étape de calcul des un ou plusieurs parmi le nombre de cellules vivantes, le nombre de cellules mortes et un taux de survie des cellules qui sont contenues dans la suspension cellulaire, est en outre basée sur la capacité par unité de cellules vivantes obtenue à l'avance et la capacité par unité de cellules mortes obtenue à l'avance.

2. Procédé de comptage de cellules vivantes selon la revendication 1, comprenant :
une étape de mesure de la capacité d'une suspension cellulaire dans laquelle le nombre de cellules vivantes est connu pour obtenir une capacité par unité de cellules vivantes ; et
une étape de mesure de la capacité d'une suspension cellulaire dans laquelle le nombre de cellules mortes est connu pour obtenir une capacité par unité de cellules mortes.

3. Procédé de comptage de cellules vivantes selon la revendication 1, dans lequel
la capacité de la suspension cellulaire est utilisée pour calcul après que la capacité de fond de substances autres que les cellules a été supprimée.

4. Procédé de comptage de cellules vivantes selon la revendication 3, comprenant :
une étape de séparation des cellules contenues dans la suspension cellulaire ; et
une étape de mesure de la capacité d'une solution sans cellule dont les cellules sont séparées.

5. Dispositif de comptage de cellules vivantes (1), comprenant :
un capteur de turbidité (2) configuré pour mesurer une quantité de matières en suspension contenue dans une suspension cellulaire ;
un instrument de mesure (3) configuré pour mesurer la capacité de la suspension cellulaire qui varie selon que les cellules soient vivantes ou mortes ; et
une unité de calcul configurée pour calculer une ou plusieurs parmi le nombre de cellules vivantes, le nombre de cellules mortes, et un taux de survie des cellules qui sont contenues dans la suspension cellulaire, sur la base de la quantité de matières en suspension, et de la capacité de la suspension cellulaire,
**caractérisé en ce que** le calcul des un ou plusieurs parmi le nombre de cellules vivantes, le nombre de cellules mortes et un taux de survie des cellules qui sont contenues dans la suspension cellulaire, est en outre basé sur la capacité par unité de cellules vivantes et la capacité par unité de cellules mortes.

6. Dispositif de comptage de cellules vivantes (1) selon la revendication 5, comprenant :
une unité de stockage configurée pour stocker des données de la capacité par unité de cellules vivantes et de la capacité par unité de cellules mortes.

7. Dispositif de comptage de cellules vivantes (1) selon la revendication 5, dans lequel
la capacité de la suspension cellulaire est utilisée pour calcul après que la capacité de fond de substances autres que les cellules a été supprimée.

8. Dispositif de comptage de cellules vivantes (1) selon la revendication 7, comprenant :
un dispositif de séparation de cellules (4) configuré pour séparer les cellules contenues dans la suspension cellulaire ; et
un instrument de mesure (5) configuré pour mesurer la capacité d'une solution sans cellule dont les cellules ont été séparées.

9. Dispositif de comptage de cellules vivantes (1) selon la revendication 8, dans lequel
des exemples d'un procédé de séparation des cellules incluent un procédé de séparation centrifuge, un procédé de filtration utilisant une membrane à fibres creuses, un procédé de filtration utilisant une membrane plate, un procédé de filtre rotatif et un procédé de sédimentation gravitationnelle.

10. Dispositif de comptage de cellules vivantes (1) selon la revendication 5, dans lequel
des exemples d'un procédé pour mesurer la quantité de matières en suspension incluent un procédé de mesure de lumière transmise, un procédé de mesure de lumière diffusée, un procédé de comparaison de lumière transmise et de lumière diffusée, un procédé de sphère d'intégration ou un procédé de comptage de particules.
